# EUROPEAN PATENT SPECIFICATION

(11) **EP 3 679 895 B1**
(45) Date of publication and mention of the grant of the patent: **12.08.2026**
(21) Application number: 20150505.4
(22) Date of filing: 30.06.2016
(51) Int. Cl.: A61F 2/24, A61F 2/95

(54) **ADJUSTABLE NOSECONE**
JUSTIERBARE KONISCHE SPITZE
ÉXTRÉMITÉ CONIQUE RÉGLABLE

(30) Priority: 02.07.2015 US 201562188223 P
(43) Date of publication of application: 15.07.2020
(62) Document number(s) of the earlier application(s) in accordance with Art. 76 EPC: 16742092.6 / 3 316 820
(73) Proprietor: Boston Scientific Scimed, Inc., Maple Grove, MN 55311-1566 (US)
(72) Inventor: CRISOTOMO, Crissly V., Folsom, CA 95630 (US); GAMARRA, Randy S., Santa Clara, CA 95051 (US)
(74) Representative: Peterreins Schley

(56) References cited:
- WO-A1-2013/070569
- WO-A1-2013/096545
- WO-A1-2014/122205
- WO-A1-2015/063118

## Description

### Technical Field

The present disclosure pertains to medical devices, and methods for manufacturing medical devices. More particularly, the present disclosure pertains to medical devices for delivering a replacement heart valve.

### Background

A wide variety of intracorporeal medical devices have been developed for medical use, for example, intravascular use. Some of these devices include guidewires, catheters, medical device delivery systems (e.g., for stents, grafts, replacement valves, etc.), and the like. These devices are manufactured by any one of a variety of different manufacturing methods and may be used according to any one of a variety of methods. Of the known medical devices and methods, each has certain advantages and disadvantages. There is an ongoing need to provide alternative medical devices as well as alternative methods for manufacturing and using medical devices.

WO 2014/122205 A1 discloses a delivery catheter for a prosthetic heart valve, the valve being expandable from a collapsed condition for delivery to an expanded condition for implantation. The delivery catheter comprises a holder for engaging the valve in the collapsed state of the valve. The holder is configured such that: (i) in response to a radial compression force exerted thereon, the holder has a first shape defining an exposed engagement region for engaging at least a portion of the collapsed prosthetic heart valve for restraining axial displacement of the prosthetic valve in at least one axial direction of the catheter, and (ii) in response to removal of the radial compression force upon expansion of the valve, at least a portion of the holder transitions to a second shape. WO 2013/096545 A1 discloses a medical device delivery system with outer sheath, inner catheter, and a tube extension that shifts between elongated and shortened configurations.

### Brief Summary

According to the present application, a medical device delivery system comprises an outer sheath, an inner catheter disposed within the outer sheath, the inner catheter including a plurality of lumens formed therein, a tubular extension extending distally from a distal end of the inner catheter, wherein the tubular extension is axially movable relative to the inner catheter, a handle attached to the outer sheath, wherein the handle is configured to shift the outer sheath between a first position and a second position relative to the inner catheter, and a medical device releasably coupled to the inner catheter, wherein, during delivery, the medical device is disposed in an elongated and low profile delivery configuration within the outer sheath distally of the inner catheter.

In addition, the medical device delivery system may further comprise a nose cone attached to the tubular extension, in particular wherein the nose cone may be fixedly attached at a distal end of the tubular extension.

In addition or alternatively, the inner catheter may be fixed to the handle.

In addition or alternatively, the tubular extension may extend through the medical device.

In addition or alternatively, the tubular extension may be fixedly attached to a hypotube slidably received within one of the plurality of lumens in the catheter.

In addition or alternatively, the medical device delivery system may further comprise a luer connector attached to a proximal end of the hypotube, the luer connector being configured to engage a proximal end of the handle.

In addition or alternatively, the luer connector, the hypotube, the tubular extension, and the nose cone together may form a nose cone subassembly having a guidewire lumen extending through the medical device delivery system, wherein the guidewire lumen may be configured to slidably receive a guidewire therein.

In addition or alternatively, the luer connector may be threadably attached to the proximal end of the hypotube.

In addition or alternatively, the luer connector may include a protrusion preventing distal axial movement of the luer connector past the proximal end of the handle.

In addition or alternatively, the nose cone subassembly may be axially movable relative to the inner catheter and the handle.

In addition or alternatively, the nose cone may be proximally movable during deployment of the medical device.

In addition or alternatively, a proximal portion of the nose cone may be configured to matingly engage a distal end of the outer sheath in the first position.

In addition or alternatively, the nose cone may be spaced distally from the outer sheath in the second position.

In addition or alternatively, the medical device may be disposed about the tubular extension.

In addition or alternatively, the tubular extension may be axially movable in a proximal direction relative to the inner catheter.

In addition or alternatively, the handle may include a control knob rotatable about a longitudinal axis of the handle, wherein the control knob may be operatively connected to the outer sheath such that rotation of the control knob translates the outer sheath axially.

In addition or alternatively, the medical device delivery system may be configured to deliver and/or deploy a replacement heart valve, a valve repair device or a valvuloplasty device.

The above summary of some embodiments is not intended to describe each disclosed embodiment or every implementation of the present invention. The Figures, and Detailed Description, which follow, more particularly exemplify these embodiments.

### Brief Description of the Drawings

Figure 1 is side view of an example valve delivery system;
Figure 2 is a partial perspective view of a portion of the example valve delivery system of Figure 1;
Figure 3 is a partial perspective view of a portion of the example valve delivery system of Figure 1;
Figure 4 is a partial cut-away side view of the example valve delivery system of Figure 1;
Figure 5 is a partial cut-away side view of the example valve delivery system of Figure 1;
Figure 6 is a partial perspective view of a portion of the example valve delivery system of Figure 1;
Figures 7-8 illustrate movement of a nosecone subassembly in a side view of the example valve delivery system of Figure 1;
Figure 9 is a side view of an example nosecone subassembly;
Figure 10 is a perspective view of a portion of the example nosecone subassembly of Figure 9; and
Figure 11 is a side view of a portion of the example nosecone subassembly of Figure 9.

While the invention is amenable to various modifications and alternative forms, specifics thereof have been shown by way of example in the drawings and will be described in detail. It should be understood, however, that the intention is not to limit the invention to the particular embodiments described. The invention relates to a medical device deliery system as defined in claim 1. Embodiments of the invention are recited in the dependent claims.

### Detailed Description

The following description should be read with reference to the drawings, which are not necessarily to scale, wherein like reference numerals indicate like elements throughout the several views. The detailed description and drawings are intended to illustrate but not limit the claimed invention. Those skilled in the art will recognize that the various elements described and/or shown may be arranged in various combinations and configurations without departing from the scope of the disclosure. The detailed description and drawings illustrate example embodiments of the claimed invention.

For the following defined terms, these definitions shall be applied, unless a different definition is given in the claims or elsewhere in this specification.

All numeric values are herein assumed to be modified by the term "about," whether or not explicitly indicated. The term "about", in the context of numeric values, generally refers to a range of numbers that one of skill in the art would consider equivalent to the recited value (i.e., having the same function or result). In many instances, the term "about" may include numbers that are rounded to the nearest significant figure. Other uses of the term "about" (i.e., in a context other than numeric values) may be assumed to have their ordinary and customary definition(s), as understood from and consistent with the context of the specification, unless otherwise specified.

The recitation of numerical ranges by endpoints includes all numbers within that range, including the endpoints (e.g. 1 to 5 includes 1, 1.5, 2, 2.75, 3, 3.80, 4, and 5).

Although some suitable dimensions, ranges and/or values pertaining to various components, features and/or specifications are disclosed, one of skill in the art, incited by the present disclosure, would understand desired dimensions, ranges and/or values may deviate from those expressly disclosed.

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

Relative terms such as "proximal", "distal", "advance", "retract", variants thereof, and the like, may be generally be considered with respect to the positioning, direction, and/or operation of various elements relative to a user/operator/manipulator of the device, wherein "proximal" and "retract" indicate or refer to closer to or toward the user and "distal" and "advance" indicate or refer to farther from or away from the user. Other relative terms, such as "upstream" and "downstream" refer to a direction of fluid flow within a lumen, such as a body lumen or blood vessel.

It is noted that references in the specification to "an embodiment", "some embodiments", "other embodiments", etc., indicate that the embodiment(s) described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it would be within the knowledge of one skilled in the art to effect such feature, structure, or characteristic in connection with other embodiments, whether or not explicitly described, unless clearly stated to the contrary. That is, the various individual elements described below, even if not explicitly shown in a particular combination, are nevertheless contemplated as being combinable or arrangable with each other to form other additional embodiments or to complement and/or enrich the described embodiment(s), as would be understood by one of ordinary skill in the art.

For the purpose of clarity, certain identifying numerical nomenclature (e.g., first, second, third, fourth, etc.) may be used throughout the description and/or claims to name and/or differentiate between various described and/or claimed features. It is to be understood that the numerical nomenclature is not intended to be limiting and is exemplary only. In some embodiments, alterations of and deviations from previously-used numerical nomenclature may be made in the interest of brevity and clarity. That is, a feature identified as a "first" element may later be referred to as a "second" element, a "third" element, etc. or may be omitted entirely, and/or a different feature may be referred to as the "first" element. The meaning and/or designation in each instance will be apparent to the skilled practitioner.

Diseases and/or medical conditions that impact the cardiovascular system are prevalent in the United States and throughout the world. Traditionally, treatment of the cardiovascular system was often conducted by directly accessing the impacted part of the system. For example, treatment of a blockage in one or more of the coronary arteries was traditionally treated using coronary artery bypass surgery. As can be readily appreciated, such therapies are rather invasive to the patient and require significant recovery times and/or treatments. More recently, less invasive therapies have been developed, for example, where a blocked coronary artery could be accessed and treated via a percutaneous catheter (e.g., angioplasty). Such therapies have gained wide acceptance among patients and clinicians.

Some relatively common medical conditions may include or be the result of inefficiency, ineffectiveness, or complete failure of one or more of the valves within the heart. For example, failure of the aortic valve can have a serious effect on a human and could lead to serious health condition and/or death if not dealt with. Treatment of defective heart valves poses other challenges in that the treatment often requires the repair or outright replacement of the defective valve. Such therapies may be highly invasive to the patient. Disclosed herein are medical devices that may be used for delivering a medical device to a portion of the cardiovascular system in order to diagnose, treat, and/or repair the system. At least some of the medical devices disclosed herein may be used to deliver and implant a replacement heart valve (e.g., a replacement aortic valve). In addition, the devices disclosed herein may deliver the replacement heart valve percutaneously and, thus, may be much less invasive to the patient. The devices disclosed herein may also provide a number of additional desirable features and benefits as described in more detail below.

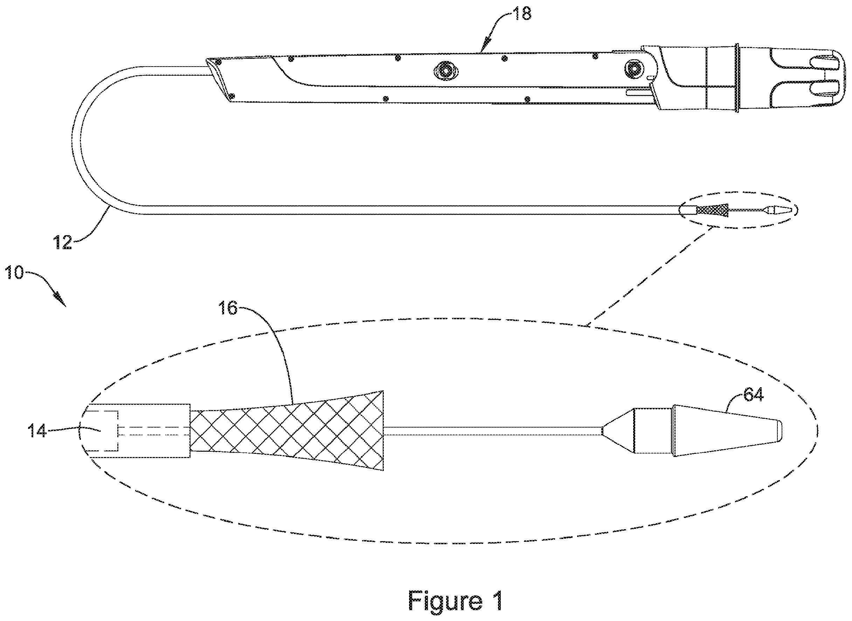

Figure 1 is a side view of an example valve delivery system 10. It should be noted that some features of the valve delivery system 10 are either not shown, or are shown schematically, in Figure 1 for simplicity. Additional details regarding some of the components of the valve delivery system 10 are provided in other figures in greater detail. The valve delivery system 10 may be used to deliver and/or deploy a variety of medical devices to a number of locations within the anatomy. In at least some embodiments, the valve delivery system 10 is a replacement heart valve delivery system (e.g., a replacement aortic valve delivery system) that can be used for percutaneous delivery of a replacement heart valve. This, however, is not intended to be limiting as the valve delivery system 10 may also be used for other interventions including mitral valve replacement, valve repair, valvuloplasty, and the like, or other similar interventions.

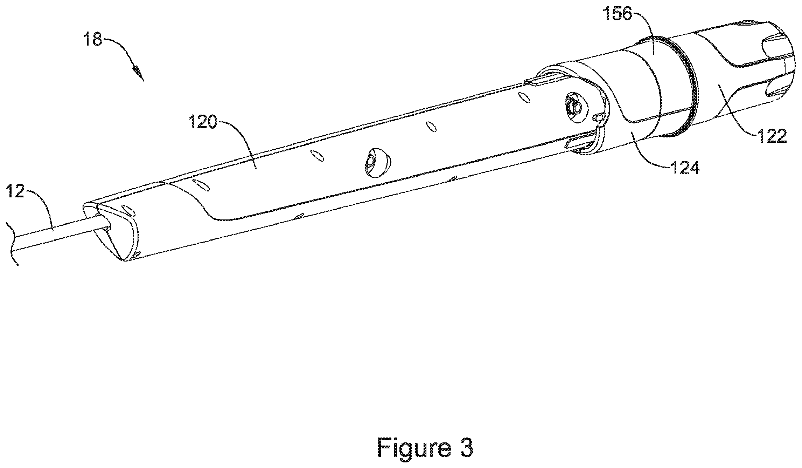

According to the present invention, the valve delivery system 10 is generally described as a catheter system that includes an outer sheath 12 and an inner catheter 14 (a portion of which is shown in Figure 1 in phantom line) disposed within and/or extending at least partially through the outer sheath 12. A valve replacement implant 16 is releasably coupled to the inner catheter 14 and disposed within the outer sheath 12 during delivery of the valve replacement implant 16. A device handle 18 is disposed at, attached to, and/or coupled to a proximal end of the outer sheath 12 and the inner catheter 14, as seen in Figures 3-5 for example. The outer sheath 12 is axially translatable relative to the device handle 18 and/or the inner catheter 14. In some embodiments, the inner catheter 14 may be fixedly attached to and/or fixed in position relative to the device handle 18. In general, the device handle 18 is configured to shift (i.e., translate, move, reposition, etc.) the outer sheath 12 between a first position and a second position relative to the inner catheter 14, as well as aid in the deployment of the valve replacement implant 16.

In use, the valve delivery system 10 may be advanced percutaneously through a patient's vasculature to a position adjacent to an area of interest. For example, valve delivery system 10 may be advanced through the vasculature to a position adjacent to a defective aortic valve. During delivery, the valve replacement implant 16 is generally disposed in an elongated and low profile "delivery" configuration at, coupled to, and/or within the outer sheath 12 distally of the inner catheter 14, wherein the outer sheath 12 is disposed at a first position relative to the inner catheter. Once positioned, the device handle 18 may be actuated to proximally retract the outer sheath 12 to a second position relative to the inner catheter 14, which may be held stationary by the device handle 18, to expose the valve replacement implant 16. The valve replacement implant 16 may be actuated in order to expand implant into a generally shortened and larger profile "deployed" configuration suitable for implantation within the anatomy. After the valve replacement implant 16 is suitably deployed within the anatomy, the valve replacement implant 16 may be released and/or detached from the valve delivery system 10, and the valve delivery system 10 may be removed from the vasculature, leaving the valve replacement implant 16 in place to function as, for example, a suitable replacement for the native aortic valve. In at least some interventions, the valve replacement implant 16 may be deployed within the native aortic valve (e.g., the native valve is left in place and not excised). Alternatively, the native aortic valve may be removed (e.g., such as through valvuloplasty, for example) and the valve replacement implant 16 may be deployed in its place as a replacement.

In some embodiments, the outer sheath 12 may define a proximal portion and a distal portion. In some embodiments, the distal portion may have a slightly enlarged or flared inner diameter, which may provide additional space for holding the medical device implant therein. In some embodiments, an inner diameter of the outer sheath 12 along the proximal portion may be in the range of about 0.254 to 1.27 cm (0.10 to 0.50 inches), or about 0.508 to 1.016 cm (0.20 to 0.40 inches), or about 0.508 to 0.762 cm (0.20 to 0.30 inches), or about 0.56388 ± 0.0508 cm (0.222 ± 0.002 inches). In some embodiments, an inner diameter of the outer sheath 12 along the distal portion may be in the range of about 0.254 to 1.27 cm (0.10 to 0.50 inches), or about 0.508 to 1.016 cm (0.20 to 0.40 inches), or about 0.508 to 0.762 cm (0.20 to 0.30 inches), or about 0.579 to 0.5842 cm (0.228 to 0.230 inches). In some embodiments, at the distal end of the distal portion may be a distal tip, which may be flared or otherwise have a funnel-like shape. The funnel-like shape may increase the outer diameter (and the inner diameter) of the outer sheath 12 at the distal tip and in some embodiments may aid in the sheathing and/or re-sheathing of the medical device implant into the outer sheath 12. In some embodiments, other than at the distal tip, the outer sheath 12 may have a generally constant outer diameter. For example, the outer sheath 12 may have an outer diameter in the range of about 0.254 to 1.27 cm (0.10 to 0.50 inches), or about 0.508 to 1.016 cm (0.20 to 0.40 inches), or about 0.508 to 0.762 cm (0.20 to 0.30 inches), or about 0.6858 cm (0.270 inches). These are just examples. Other embodiments are contemplated that have differing dimensions (including those appropriate for differently sized patients including children) and/or arrangements for the outer diameter and/or the inner diameter of the outer sheath 12. These contemplated embodiments include delivery sheaths with flared or otherwise variable outer diameters, embodiments with constant inner diameters, combinations thereof, and the like. In some embodiments, the outer sheath 12 may also have a length that is appropriate for reaching the intended area of interest within the anatomy. For example, in some embodiments, the outer sheath 12 may have a length in the range of about 30 to 200 cm, or about 60 to 150 cm, or about 100 to 120 cm, or about 108 ± 0.20 cm. In some embodiments, the outer sheath 12 and/or one or more portions of the outer sheath 12 may be curved. For example, in some embodiments, the distal portion of the outer sheath 12 may be curved. In one example, a radius of the curved distal portion (as measured from a central axis of the outer sheath 12) may be in the range of about 2 to 6 cm (20 to 60 mm), or about 3 to 4 cm (30 to 40 mm), or about 3.675 cm (36.75 mm). Again, these dimensions are examples and are not intended to be limiting.

In some embodiments, the outer sheath 12 may be formed from a singular monolithic tube or unitary member. Alternatively, the outer sheath 12 may include a plurality of layers or portions. One or more of these layers may include a reinforcing structure such as a braid, coil, mesh, combinations thereof, or the like. For example, in some embodiments, the outer sheath 12 may include an inner liner or layer. In some embodiments, an intermediate or tier layer may be disposed on the inner liner. In some embodiments, a reinforcement may be disposed on the intermediate layer. In some embodiments, a topcoat or outer layer may be disposed on or over the reinforcement. In some embodiments, an outer coating (e.g., a lubricious coating, a hydrophilic coating, a hydrophobic coating, etc.) may be disposed on, over, and/or along portions or all of the topcoat or outer layer. These are just examples. Several alternative structural configurations are contemplated for the outer sheath 12 including embodiments including two or more layers that may be different, embodiments without a reinforcement, and the like, or other suitable configurations.

The dimensions and materials utilized for the various layers of the outer sheath 12 may also vary. For example, an inner liner may include a polymeric material such as fluorinated ethylene propylene (FEP) and may have a thickness in the range of about 0.00254 to 0.0127 cm (0.001 to 0.005 inches) or about 0.00762 ± 0.00254 (0.003 ± 0.001 inches), an intermediate layer may include a polymer material such as polyether block amide (e.g., PEBAX 6333) and may have a thickness in the range of about 0.00254 to 0.0127 cm (0.001 to 0.005 inches) or about 0.00508 ± 0.00254 (0.002 ± 0.001 inches), an outer coating may include a polymer material such as polyether block amide (e.g., PEBAX 7233) and may have a thickness in the range of about 0.00254 to 0.0254 cm (0.001 to 0.01 inches). In some embodiments, an outer coating may vary in thickness. For example, along a proximal portion, an outer coating may have greater thickness, such as about 0.0127 to about 0.0508 cm or about 0.02159 cm (0.005 to 0.02 inches or about 0.0085 inches), than along a distal portion and/or at a distal tip, which may be about 0.0127 to about 0.0508 cm or about 0.01651 cm (e.g., about 0.005 to 0.02 inches or about 0.0065 inches). These are just examples as other suitable materials may be used.

A reinforcement may also vary in form. In at least some embodiments, a reinforcement may take the form of a braid, coil, mesh, or the like. For example, in some embodiments, a reinforcement may include a metallic braid (e.g., stainless steel). In some of these embodiments, a reinforcement may also include additional structures such as one or more longitudinally-extending strands. For example, a reinforcement may include a pair of longitudinally-extending aramid and/or para aramid strands (for example, KEVLAR^{®}) disposed on opposite sides of the braid. In some embodiments, these strands may or may not be woven into portions or all of the braid.

In some embodiments, a distal end region of the inner catheter 14 may include a step in outer diameter that defines a decreased outer diameter section. For example, a decreased outer diameter section may have an outer diameter in the range of about 0.127 to 0.635 cm (0.05 to 0.25 inches), or about 0.254 to 0.508 cm (0.10 to 0.20 inches), or about 0.38608 ± 0.00762 (0.152 ± 0.003 inches) as opposed to the remainder of the inner catheter 14 where the outer diameter may be in the range of about 0.127 to 0.762 cm (0.05 to 0.30 inches), or about 0.254 to 0.635 cm (0.10 to 0.25 inches), or about 0.508 ± 0.0254 cm (0.20 ± 0.01 inches). The decreased outer diameter section may define a region where other components of the valve delivery system 10 may be attached.

In some embodiments, the inner catheter 14 may include a plurality of lumens formed therein. In some embodiments, the inner catheter 14 may be formed as an extruded polymeric shaft. Other forms are also contemplated including other polymer shafts or tubes, metallic shafts or tubes, reinforced shafts or tubes, or the like including other suitable materials such as those disclosed herein. In some embodiments, the polymeric shaft may be a singular monolithic or unitary member. In some embodiments, the polymeric shaft may include a plurality of portions or segments that are coupled together. The total length of the inner catheter 14 and/or the polymeric shaft may be in the range of about 60 to 150 cm, or about 80 to 120 cm, or about 100 to 115 cm, or about 112 ± 0.02 cm. Just like the outer sheath 12, in some embodiments, the inner catheter 14 and/or the polymeric shaft may be curved, for example adjacent to a distal end thereof. In some embodiments, the polymeric shaft may have one or more sections with a differing hardness/stiffness (e.g., differing shore durometer). For example, the polymeric shaft may have a proximal region and an intermediate region. In some embodiments, the proximal region may include a generally stiff polymeric material such as a 72D polyether block amide (e.g., 72D PEBAX) and may have a length in the range of about 60 to 150 cm, or about 80 to 120 cm, or about 100 to 115 cm, or about 109.5 ± 0.02 cm. In some embodiments, the intermediate region may include a 40D polyether block amide (e.g., 40D PEBAX) and may have a length in the range of about 5 to 25 mm, or about 10 to 20 mm, or about 15 ± 0.01 mm. The decreased outer diameter section may also differ from the proximal region and/or the intermediate region and, in some embodiments, may include a 72D polyether block amide (e.g., 72D PEBAX) and may have a length in the range of about 0.5 to 2 cm (5 to 20 mm), or about 0.8 to 1.5 cm (8 to 15 mm), or about 1 ± 0.001 cm (10 ± 0.01 mm). These are just examples. Other suitable polymers may also be used, such as, but not limited to, other polymers disclosed herein.

According to the present invention, the inner catheter 14 includes a plurality of lumens extending at least partially therethrough. For example, in some embodiments, the inner catheter 14 and/or the polymeric shaft may include a first lumen, a second lumen, a third lumen, and/or a fourth lumen. In general, the lumens extend along the entire length of the inner catheter 14 and/or the polymeric shaft. Other embodiments are contemplated, however, where one or more of lumens extend along only a portion of the length of inner catheter 14 and/or the polymeric shaft. For example, the fourth lumen may stop just short of the distal end of the inner catheter 14 and/or the polymeric shaft and/or be filled in at its distal end to effectively end the fourth lumen proximal of the distal end of the inner catheter 14 and/or the polymeric shaft.

Disposed within one of the lumens (e.g., a first lumen) may be one or more push-pull rods (not shown), which are used to expand and/or elongate the medical device implant when the device handle 18 is actuated by connecting a portion of the device handle 18 to the medical device implant to transmit rotational and/or axial motion thereto. In some embodiments, one or more of the lumens (e.g., the first lumen) may be lined with a low friction liner (e.g., a FEP liner). Disposed within a second lumen may be a pin release mandrel (not shown), which in some embodiments may facilitate release of the valve replacement implant 16. In some embodiments, the second lumen may be lined with a hypotube liner. In some embodiments, a third lumen may be a guidewire lumen. In some embodiments, the third lumen may be lined with a hypotube liner. In some embodiments, a fourth lumen may be used to house a non-stretch wire or member. The form of the non-stretch wire or member may vary. In some embodiments, the non-stretch wire or member may take the form of a stainless steel braid. The non-stretch wire or member may optionally include a pair of longitudinally-extending aramid and/or para aramid strands (for example, KEVLAR^{®}) disposed on opposite sides of the braid. In general, rather than being "disposed within" the fourth lumen, the non-stretch wire or member may be embedded within the fourth lumen and/or the polymeric shaft. In addition, the non-stretch wire or member may extend to a position adjacent to the distal end portion but not fully to the distal end of the inner catheter 14 and/or the polymeric shaft. For example, a short distal segment of the fourth lumen may be filled in with polymer material adjacent to the distal end of inner catheter 14 and/or the polymeric shaft.

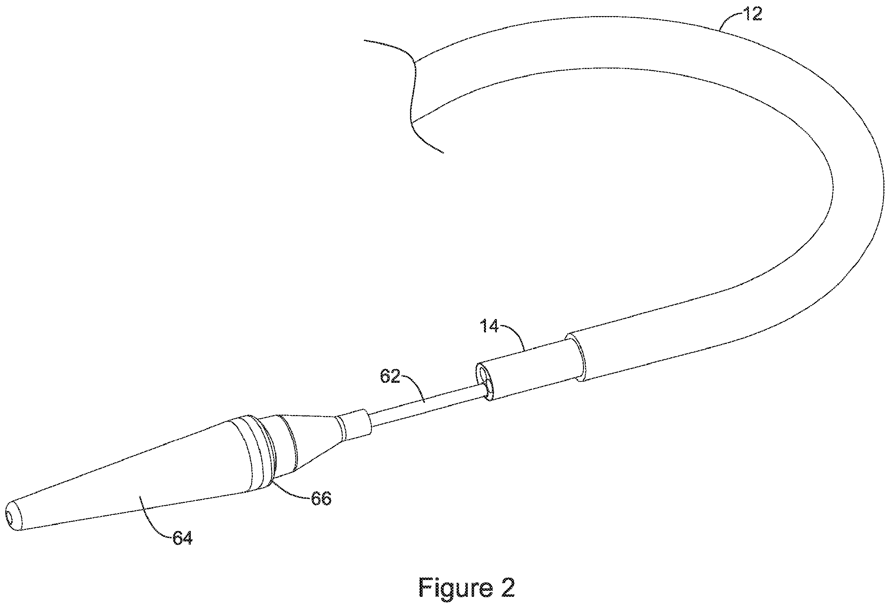

In some embodiments, the valve delivery system 10 may include a tubular extension 62 extending distally from a distal end of the inner catheter 14, as seen in Figure 2 for example. In some embodiments, the tubular extension 62 may be axially movable and/or translatable relative to the inner catheter 14. In some embodiments, a nose cone 64 may be fixedly attached to the tubular extension 62 at a distal end of the tubular extension 62. The nose cone 64 generally may be designed to have an atraumatic shape. In some embodiments, a proximal portion of the nose cone 64 may be configured to matingly engage a distal end of the outer sheath in the first position, and in some embodiments, the nose cone 64 may include a ridge 66 that is configured to abut the distal end or distal tip of the outer sheath 12 in the first position, such as during delivery of the valve replacement implant 16 for example. As readily seen in the Figures, in some embodiments, the valve replacement implant 16 may be disposed about and/or may surround the tubular extension 62.

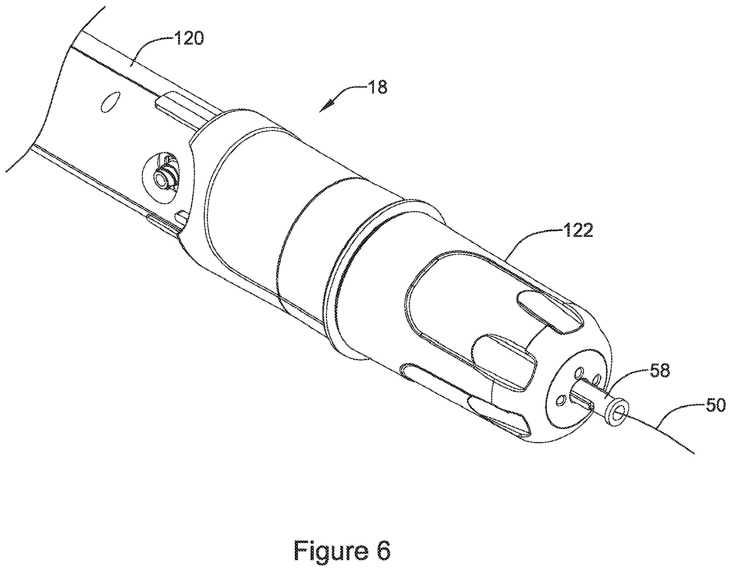

In some embodiments, the tubular extension 62 may be fixedly attached to a hypotube 60 slidably received within one of the plurality of lumens of the inner catheter 14. In some embodiments, a luer connector 58 may be attached to a proximal end of the hypotube 60. In some embodiments, the luer connector 58 may be configured to engage a proximal end of the device handle 18, as seen in Figure 6 for example. In some embodiments, the luer connector 58, the hypotube 60, the tubular extension 62, and the nose cone 64 may together form a nose cone subassembly having a guidewire lumen extending therethrough, and thus may define a guidewire lumen extending through the percutaneous valve delivery system 10. In some embodiments, the guidewire lumen may be configured to slidably receive a guidewire 50 therein, as seen in Figures 6-8.

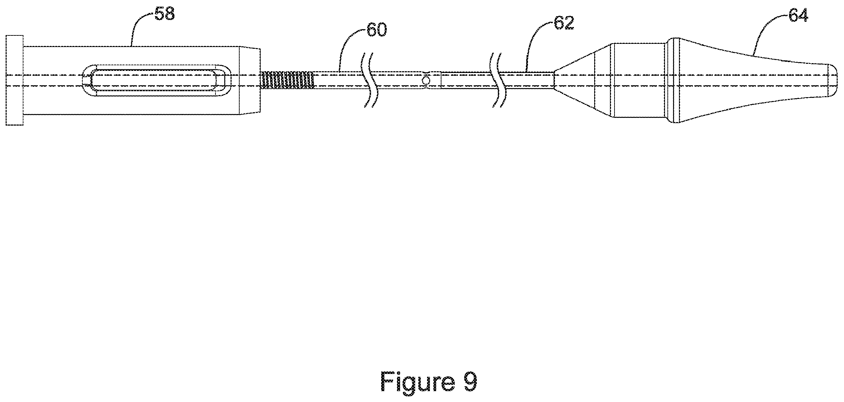

Figure 9 illustrates an example nose cone subassembly including a luer connector 58, a hypotube 60, a tubular extension 62, and a nose cone 64, the nose cone subassembly having a guidewire lumen extending therethrough. In some embodiments, the luer connector 58 may be threadably attached to the proximal end of the hypotube 60. In some embodiments, a distal end of the hypotube 60 may be fixedly attached to a proximal end of the tubular extension 62, such that an axial lumen of the hypotube 60 is in fluid communication with an axial lumen of the tubular extension 62, thereby forming at least a central portion of the guidewire lumen of the nose cone subassembly. In some embodiments, the nose cone subassembly may be axially movable, translatable, and/or actuatable relative to the inner catheter 14 and/or the device handle 18. In some embodiments, the nose cone 64 may include a lumen extending therethrough and in fluid communication with the lumen of the tubular extension 62 so as to form a distal portion of the guidewire lumen.

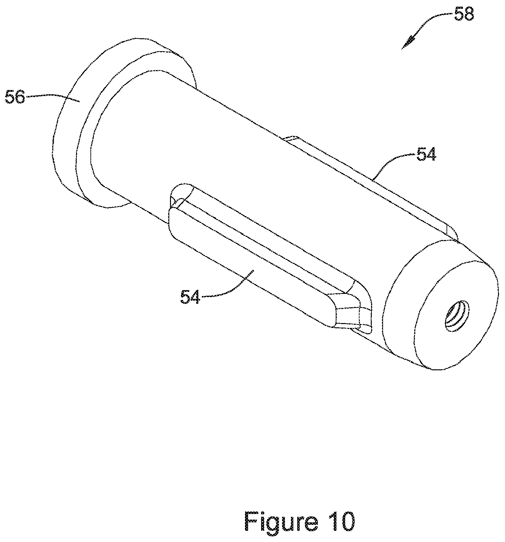

Figure 10 illustrates an example luer connector 58 having a generally elongated tubular body. In some embodiments, the luer connector 58 may include a protrusion 56 disposed at or adjacent to, and extending radially outward from, a proximal end of the luer connector 58. In some embodiments, the protrusion 56 may be an annular or ring-like protrusion. In at least some embodiments, the protrusion 56 may prevent distal axial movement of the luer connector 58, and therefore the nose cone subassembly to which the luer connector 58 is attached, past the proximal end of the device handle 18 and/or the rotatable control knob 122. In some embodiments, the protrusion 56 may be configured to engage with and/or abut the proximal end of the device handle 18 and/or a corresponding mating recess in the proximal end of the device handle 18 and/or the rotatable control knob 122. In some embodiments, the luer connector 58 may include one or more guide bars 54 extending generally parallel to a central axis of the luer connector 58 and extending laterally outward from an outer surface of the elongated tubular body of the luer connector 58. In some embodiments, the one or more guide bars 54 may permit the luer connector 58 to slidingly engage the device handle 18 while preventing rotational movement of the luer connector 58 relative to the device handle 18 when the one or more guide bars 54 is engaged with the device handle 18. In some embodiments, the one or more guide bars 54 may permit the luer connector 58 to slidingly engage the device handle 18 while also permitting rotational movement of the luer connector 58 relative to the device handle 18 when the one or more guide bars 54 is engaged with the device handle. In some embodiments, the one or more guide bars 54 may form a helical or spiral configuration enabling the luer connector 58 to be removably threaded or screwed into the proximal end of the device handle 18 to prevent axial movement or translation of the nose cone subassembly when the luer connector 58 is engaged with the device handle 18.

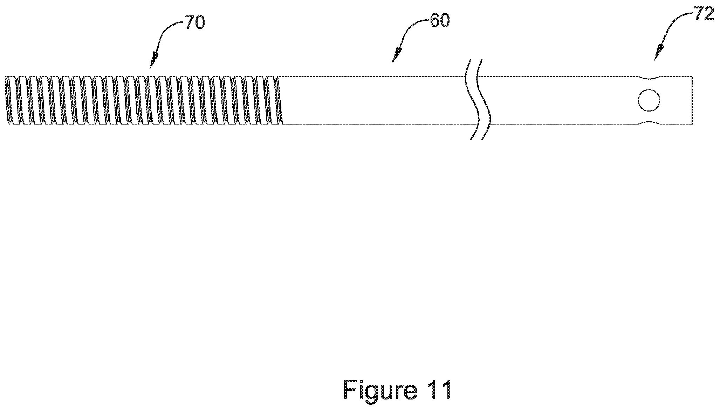

Figure 11 illustrates an example hypotube 60. In some embodiments, the hypotube 60 may include a lumen extending therethrough. In some embodiments, the hypotube 60 may include an externally threaded proximal end 70 configured to threadably engage an internally threaded lumen of the luer connector 58. In some embodiments, the lumen of the hypotube 60 may be in fluid communication with the lumen of the luer connector 58 so as to form a proximal portion of the guidewire lumen of the nose cone subassembly. In some embodiments, the hypotube 60 may include a plurality of apertures 72 extending laterally through a side wall of the hypotube 60 adjacent to a distal end of the hypotube 60. In some embodiments, the plurality of apertures 72 may be regularly or evenly spaced about the circumference of the hypotube 60. In some embodiments, the plurality of apertures 72 may be irregularly or unevenly spaced about the circumference of the hypotube 60. In at least some embodiments, the plurality of apertures may be configured to engage with and/or receive one or more portions of the tubular extension 62 therein to form a mechanical connection fixedly attaching the hypotube 60 to the tubular extension 62. In some embodiments, each of the plurality of apertures may engage or receive one portion of the tubular extension 62 extending radially outward from an outer surface of the tubular extension 62. In some embodiments, the lumen of the hypotube 60 may be in fluid communication with a lumen of the tubular extension 62 so as to form a central portion of the guidewire lumen of the nose cone subassembly.

As can be seen in Figures 3-5, the device handle 18 includes a handle housing 120. In some embodiments, the device handle 18 may also include a rotatable control knob 122 disposed on or about the handle housing 120 (e.g., at a proximal end of the handle housing 120) and may be used to move one or more of the components of the valve delivery system 10 (e.g., the outer sheath 12, the push-pull rods, the pin release mandrel, etc.). In some embodiments, the device handle 18 may include a rotatable collar 156 disposed about the handle housing 120. In some embodiments, the rotatable collar 156 may be disposed adjacent the rotatable control knob 122. In some embodiments, the rotatable control knob 122 may be disposed about a proximal portion of the rotatable collar 156. In some embodiments, a slidable door 124 may also be disposed about the handle housing 120. In some embodiments, the slidable door 124 may translate distally to expose a distal portion of the rotatable collar 156 which may be positioned generally under the slidable door 124. In some embodiments, the rotatable collar 156 may be rotated to move one or more components of the valve delivery system 10. In some embodiments, the device handle 18 may also include one or more apertures and/or flush ports that can be used to flush the valve delivery system 10. In some embodiments, a distal flush port and a proximal flush port may be accessible from an exterior of the handle housing 120 through a distal aperture and a proximal aperture, respectively.

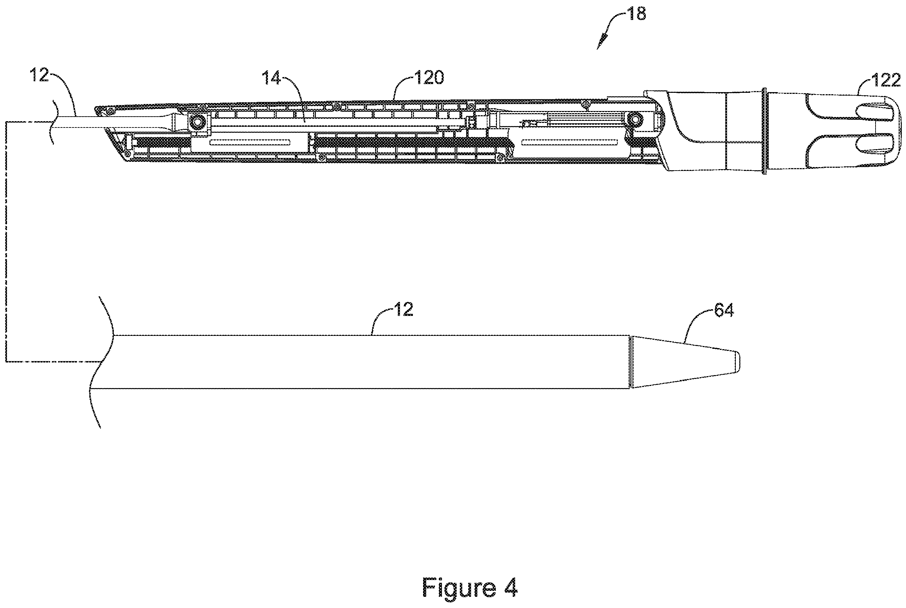

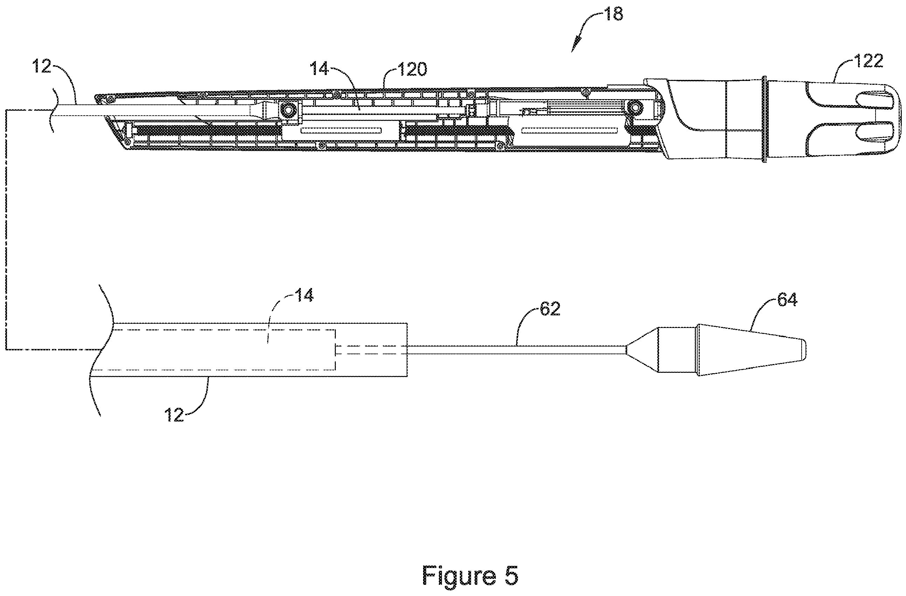

Figures 4 and 5 illustrate a side view of the device handle 18 with a portion of the handle housing 120 removed, exposing at least some of the interior components. Here it can be seen that the outer sheath 12 may be fixedly attached to a sheath adapter. The sheath adapter may be attached to a sheath carriage, which may be threaded onto a lead screw. The distal flush port may be disposed on the sheath adapter. In general, the distal flush port provides access to the interior or lumen of the outer sheath 12 (e.g., access to space between the inner catheter 14 and the outer sheath 12) so that a clinician can flush fluid through the lumen of the outer sheath 12 to remove any unwanted materials (e.g., air, fluid, contaminants, etc.) therein prior to use of the valve delivery system 10. In at least some embodiments, the distal flush port has a luer type connector (e.g., a one-way luer connector) that allows a device such as a syringe with a corresponding connector to be attached thereto for flushing.

Extending through and proximally from the sheath adapter is the inner catheter 14. A proximal end of the inner catheter 14 is selectively attached (e.g., releasably locked) to a diverter block. The diverter block may be attached to a support body. The proximal flush port may be disposed on the support body and can be used to flush the lumen(s) of the inner catheter 14 and/or the polymeric shaft, and may function similarly to the distal flush port, for example. In general, the diverter block and/or the support body may have one or more passageways or lumens formed therein.

In some embodiments, the push-pull rods and/or the pin release mandrel may extend from a mechanism operatively connected to the rotatable control knob 122, the slidable door 124, and/or the rotatable collar 156 through respective passageways or lumens in the diverter block and/or the support body and into the lumen(s) of the inner catheter 14 and/or the polymeric shaft. Alternatively, the proximal ends of the push-pull rods and/or the pin release mandrel may each be attached to a shaft or hypotube (e.g., solid in cross-section, tubular, etc.), and each of the shafts or hypotubes may extend proximally therefrom through one of the one or more passageways or lumens to the mechanism operatively connected to the rotatable control knob 122, the slidable door 124, and/or the rotatable collar 156. For example, a first shaft or hypotube and a second shaft or hypotube may extend through the passageways or lumens in the diverter block, and in some embodiments, the first shaft or hypotube may extend through a first passageway or lumen and the second shaft or hypotube may extend through a second passageway or lumen that is separate or distinct from the first passageway or lumen. In at least some embodiments, the first shaft may be attached to the pin release mandrel. In at least some embodiments, the second shaft may be attached to the push-pull rods. It should be noted that at in least some embodiments of the valve delivery system 10, three push-pull rods are utilized. In these embodiments, the three push-pull rods may come together (e.g., brought into contact with one another or otherwise brought into relatively close proximity with one another) adjacent to the distal end of the inner catheter 14 and enter the first passageway or lumen. At one or more positions along their length, the push-pull rods may be fixedly attached to one another. For example, in some embodiments, the push-pull rods may be welded together about 10.16 cm (about 4.00 inches) proximally from their distal ends. In some embodiments, the push-pull rods may be welded together proximate their proximal ends in addition to or instead of the distal weld. Proximally thereafter, the push-pull rods may extend to the second shaft.

The device handle 18 is generally configured for coordinated movement of multiple structures of the valve delivery system 10. For example, the device handle 18 is configured to allow a user to move the outer sheath 12 (e.g., relative to the inner catheter 14), move the push-pull rods, and move the pin release mandrel. Moreover, the device handle 18 is configured so that the appropriate structure can be moved at the appropriate time during the intervention so that the valve replacement implant 16 can be delivered in an efficient manner.

To help facilitate the coordinated movement, the device handle 18 may include one or more mechanisms disposed therein which may translate, transfer, and/or convert motion (e.g., rotational motion) of the rotatable control knob 122, the slidable door 124, and/or the rotatable collar 156 into axial motion, movement, and/or translation at a distal end of the valve delivery system 10.

Figures 4 and 5 partially illustrate some of the coordinated motion achieved by the device handle 18. It should be noted that some elements of the valve delivery system 10 are not shown in Figures 4 and 5 for clarity. For example, Figure 4 illustrates a first position of the outer sheath 12 relative to inner catheter 14, wherein the nose cone 64 matingly engages a distal end of the outer sheath 12 to fully sheath (e.g., contain) the valve replacement implant 16 within the outer sheath 12 in a delivery configuration. While in this position, the sheath carriage (with the distal flush port) is positioned adj acent to the distal end of the device handle 18. Upon rotation of the rotatable control knob 122 (e.g., in the clockwise direction), the lead screw begins to rotate, causing the sheath carriage to move along the lead screw in the proximal direction, resulting in proximal movement of the outer sheath 12 toward a second position relative to the inner catheter 14 and/or the nose cone 64 (e.g., "unsheathing" the valve replacement implant 16), as seen in Figure 5 for example.

Eventually, sufficient actuation of the rotatable control knob 122, the slidable door 124, and/or the rotatable collar 156, and the operatively connected mechanism(s) of the device handle 18, may actuate the valve replacement implant 16 from the delivery configuration to the deployed configuration, and may further release and/or detach the valve replacement implant 16 from the inner catheter 14 and/or the valve delivery system 10.

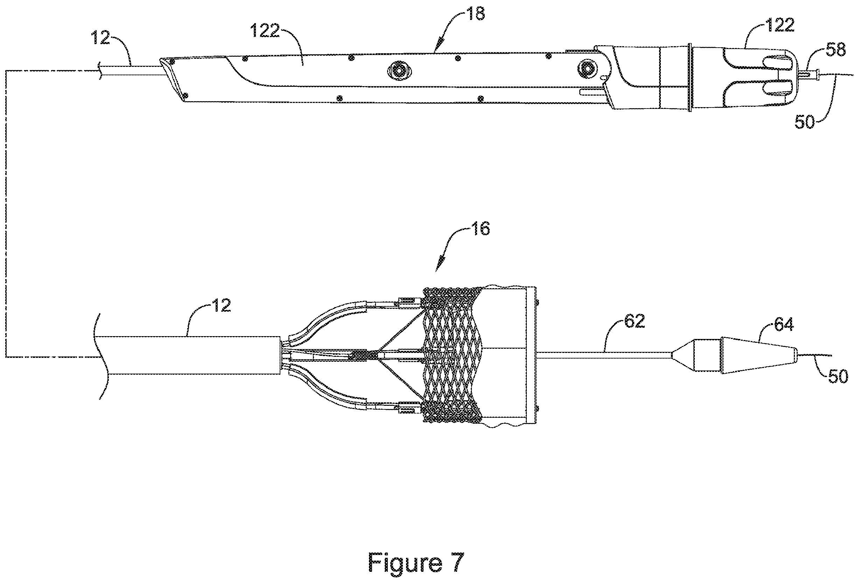

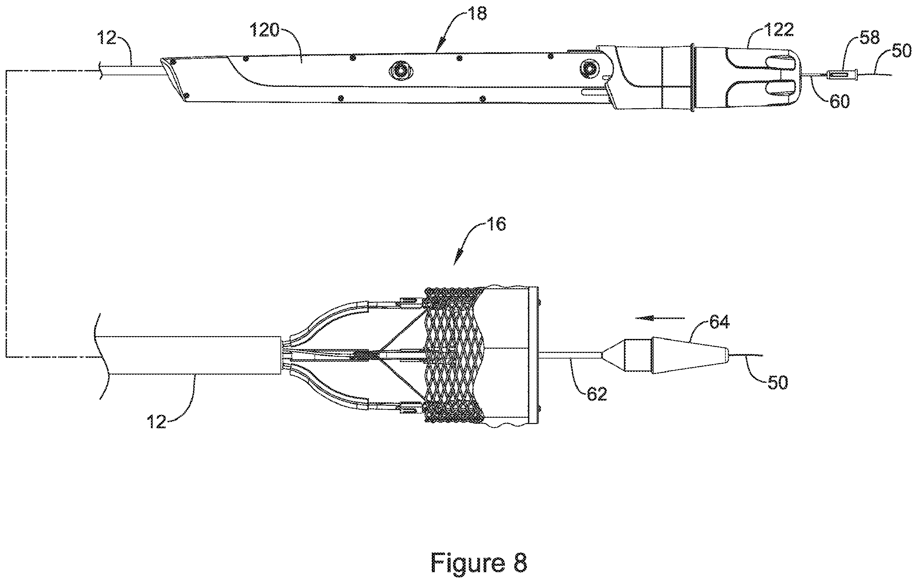

In some cases, during deployment of the valve replacement implant 16, a physician or user of the valve delivery system 10 may find a reason or desire to withdraw the nose cone 64 proximally (i.e., away from a wall of the ventricle and closer to the inner catheter 14). Since the nose cone subassembly may be slidably received within a lumen of the inner catheter 14, and the inner catheter is fixed relative to the device handle 18, proximal withdrawal of the luer connector 58 relative to the device handle 18 will cause a commensurate proximal movement of the nose cone 64 relative to the valve replacement implant 16, as seen in Figures 7 and 8 for example.

Following deployment of the valve replacement implant 16, the rotatable control knob 122 may be rotated to move the sheath carriage distally within the handle housing 120, thereby moving the outer sheath 12 from the second position relative to the inner catheter 14 distally to the first position relative to the inner catheter 14 so as to cover or re-sheath the elements of the valve delivery system 10 disposed at the distal end. The valve delivery system 10 may then be removed from the patient's anatomy.

The materials that can be used for the various components of the valve delivery system 10 (and/or other systems and components disclosed herein) and the various tubular members disclosed herein may include those commonly associated with medical devices. For example, in some embodiments, the outer sheath 12, the inner catheter 14, and/or the hypotube 60, (as well as other components disclosed herein) may be made from a metal, metal alloy, polymer (some examples of which are disclosed below), a metal-polymer composite, ceramics, combinations thereof, and the like, or other suitable material. Some examples of suitable metals and metal alloys include stainless steel, such as 304V, 304L, and 316LV stainless steel; mild steel; nickel-titanium alloy such as linear-elastic and/or super-elastic nitinol; other nickel alloys such as nickel-chromium-molybdenum alloys (e.g., UNS: N06625 such as INCONEL^{®} 625, UNS: N06022 such as HASTELLOY^{®} C-22^{®}, UNS: N10276 such as HASTELLOY^{®} C276^{®}, other HASTELLOY^{®} alloys, and the like), nickel-copper alloys (e.g., UNS: N04400 such as MONEL^{®} 400, NICKELVAC^{®} 400, NICORROS^{®} 400, and the like), nickel-cobalt-chromium-molybdenum alloys (e.g., UNS: R30035 such as MP35-N^{®} and the like), nickel-molybdenum alloys (e.g., UNS: N10665 such as HASTELLOY^{®} ALLOY B2^{®}), other nickel-chromium alloys, other nickel-molybdenum alloys, other nickel-cobalt alloys, other nickel-iron alloys, other nickel-copper alloys, other nickel-tungsten or tungsten alloys, and the like; cobalt-chromium alloys; cobalt-chromium-molybdenum alloys (e.g., UNS: R30003 such as ELGILOY^{®}, PHYNOX^{®}, and the like); platinum enriched stainless steel; titanium; combinations thereof; and the like; or any other suitable material.

As alluded to herein, within the family of commercially available nickel-titanium or nitinol alloys, is a category designated "linear elastic" or "non-super-elastic" which, although may be similar in chemistry to conventional shape memory and super elastic varieties, may exhibit distinct and useful mechanical properties. Linear elastic and/or non-super-elastic nitinol may be distinguished from super elastic nitinol in that the linear elastic and/or non-super-elastic nitinol does not display a substantial "superelastic plateau" or "flag region" in its stress/strain curve like super elastic nitinol does. Instead, in the linear elastic and/or non-super-elastic nitinol, as recoverable strain increases, the stress continues to increase in a substantially linear, or a somewhat, but not necessarily entirely linear relationship until plastic deformation begins or at least in a relationship that is more linear that the super elastic plateau and/or flag region that may be seen with super elastic nitinol. Thus, for the purposes of this disclosure linear elastic and/or non-super-elastic nitinol may also be termed "substantially" linear elastic and/or non-super-elastic nitinol.

In some cases, linear elastic and/or non-super-elastic nitinol may also be distinguishable from super elastic nitinol in that linear elastic and/or non-super-elastic nitinol may accept up to about 2-5% strain while remaining substantially elastic (e.g., before plastically deforming) whereas super elastic nitinol may accept up to about 8% strain before plastically deforming. Both of these materials can be distinguished from other linear elastic materials such as stainless steel (that can also can be distinguished based on its composition), which may accept only about 0.2 to 0.44 percent strain before plastically deforming.

In some embodiments, the linear elastic and/or non-super-elastic nickel-titanium alloy is an alloy that does not show any martensite/austenite phase changes that are detectable by differential scanning calorimetry (DSC) and dynamic metal thermal analysis (DMTA) analysis over a large temperature range. For example, in some embodiments, there may be no martensite/austenite phase changes detectable by DSC and DMTA analysis in the range of about -60 degrees Celsius (°C) to about 120 °C in the linear elastic and/or non-super-elastic nickel-titanium alloy. The mechanical bending properties of such material may therefore be generally inert to the effect of temperature over this very broad range of temperature. In some embodiments, the mechanical bending properties of the linear elastic and/or non-super-elastic nickel-titanium alloy at ambient or room temperature are substantially the same as the mechanical properties at body temperature, for example, in that they do not display a super-elastic plateau and/or flag region. In other words, across a broad temperature range, the linear elastic and/or non-super-elastic nickel-titanium alloy maintains its linear elastic and/or non-super-elastic characteristics and/or properties.

In some embodiments, the linear elastic and/or non-super-elastic nickel-titanium alloy may be in the range of about 50 to about 60 weight percent nickel, with the remainder being essentially titanium. In some embodiments, the composition is in the range of about 54 to about 57 weight percent nickel. One example of a suitable nickel-titanium alloy is FHP-NT alloy commercially available from Furukawa Techno Material Co. of Kanagawa, Japan. Some examples of nickel titanium alloys are disclosed in U.S. Patent Nos. 5,238,004 and 6,508,803. Other suitable materials may include ULTANIUM^{™} (available from Neo-Metrics) and GUM METAL^{™} (available from Toyota). In some other embodiments, a superelastic alloy, for example a superelastic nitinol can be used to achieve desired properties.

In at least some embodiments, portions or all of the outer sheath 12 and/or the inner catheter 14 may also be doped with, made of, or otherwise include a radiopaque material. Radiopaque materials are understood to be materials capable of producing a relatively bright image on a fluoroscopy screen or another imaging technique during a medical procedure. This relatively bright image may aid the user of the valve delivery system 10 in determining its location. Some examples of radiopaque materials can include, but are not limited to, gold, platinum, palladium, tantalum, tungsten alloy, polymer material loaded with a radiopaque filler, and the like. Additionally, other radiopaque marker bands and/or coils may also be incorporated into the design of the valve delivery system 10 to achieve the same result.

In some embodiments, a degree of Magnetic Resonance Imaging (MRI) compatibility is imparted into the valve delivery system 10. For example, the outer sheath 12 and/or the inner catheter 14, or portions thereof, may be made of a material that does not substantially distort the image and create substantial artifacts (i.e., gaps in the image). Certain ferromagnetic materials, for example, may not be suitable because they may create artifacts in an MRI image. The outer sheath 12 and/or the inner catheter 14, or portions thereof, may also be made from a material that the MRI machine can image. Some materials that exhibit these characteristics include, for example, tungsten, cobalt-chromium-molybdenum alloys (e.g., UNS: R30003 such as ELGILOY^{®}, PHYNOX^{®}, and the like), nickel-cobalt-chromium-molybdenum alloys (e.g., UNS: R30035 such as MP35-N^{®} and the like), nitinol, and the like, and others.

A sheath or covering (not shown) may be disposed over portions or all of the outer sheath 12 and/or the inner catheter 14 that may define a generally smooth outer surface for the valve delivery system 10. In other embodiments, however, such a sheath or covering may be absent from a portion of all of the valve delivery system 10, such that the outer sheath 12 and the inner catheter 14, either individually or together, may form an outer surface. The sheath or covering may be made from a polymer or other suitable material. Some examples of suitable polymers for the sheath or covering, the outer sheath 12, the inner catheter 14, the tubular extension 62, and/or other polymeric components of the valve delivery system may include polytetrafluoroethylene (PTFE), ethylene tetrafluoroethylene (ETFE), fluorinated ethylene propylene (FEP), polyoxymethylene (POM, for example, DELRIN^{®} available from DuPont), polyether block ester, polyurethane (for example, Polyurethane 85A), polypropylene (PP), polyvinylchloride (PVC), polyether-ester (for example, ARNITEL^{®} available from DSM Engineering Plastics), ether or ester based copolymers (for example, butylene/poly(alkylene ether) phthalate and/or other polyester elastomers such as HYTREL^{®} available from DuPont), polyamide (for example, DURETHAN^{®} available from Bayer or CRISTAMID^{®} available from Elf Atochem), elastomeric polyamides, block polyamide/ethers, polyether block amide (PEBA, for example available under the trade name PEBAX^{®}), ethylene vinyl acetate copolymers (EVA), silicones, polyethylene (PE), Marlex high-density polyethylene, Marlex low-density polyethylene, linear low density polyethylene (for example REXELL^{®}), polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide (for example, KEVLAR^{®}), polysulfone, nylon, nylon-12 (such as GRILAMID^{®} available from EMS American Grilon), perfluoro(propyl vinyl ether) (PFA), ethylene vinyl alcohol, polyolefin, polystyrene, epoxy, polyvinylidene chloride (PVdC), poly(styrene-b-isobutylene-b-styrene) (for example, SIBS and/or SIBS 50A), polycarbonates, ionomers, biocompatible polymers, other suitable materials, or mixtures, combinations, copolymers thereof, polymer/metal composites, and the like. In some embodiments the sheath can be blended with a liquid crystal polymer (LCP). For example, the mixture can contain up to about 6 percent LCP.

In some embodiments, the exterior surface of the valve delivery system 10 (including, for example, the exterior surface of the outer sheath 12 and the inner catheter 14) may be sandblasted, bead blasted, sodium bicarbonate-blasted, electropolished, etc. In these as well as in some other embodiments, a coating, for example a lubricious, a hydrophilic, a protective, or other type of coating may be applied over portions or all of the sheath, or in embodiments without a sheath over a portion of the outer sheath 12 and/or the inner catheter 14, or other portions of the valve delivery system 10. Alternatively, the sheath may comprise a lubricious, hydrophilic, protective, or other type of coating. Hydrophobic coatings such as fluoropolymers provide a dry lubricity which improves device handling and device exchanges. Lubricious coatings improve steerability and improve lesion crossing capability. Suitable lubricious polymers are well known in the art and may include silicone and the like, hydrophilic polymers such as high-density polyethylene (HDPE), polytetrafluoroethylene (PTFE), polyarylene oxides, polyvinylpyrolidones, polyvinylalcohols, hydroxy alkyl cellulosics, algins, saccharides, caprolactones, and the like, and mixtures and combinations thereof. Hydrophilic polymers may be blended among themselves or with formulated amounts of water insoluble compounds (including some polymers) to yield coatings with suitable lubricity, bonding, and solubility.

The coating and/or sheath may be formed, for example, by coating, extrusion, co-extrusion, interrupted layer co-extrusion (ILC), or fusing several segments end-to-end. The layer may have a uniform stiffness or a gradual reduction in stiffness from the proximal end to the distal end thereof. The gradual reduction in stiffness may be continuous as by ILC or may be stepped as by fusing together separate extruded tubular segments. The outer layer may be impregnated with a radiopaque filler material to facilitate radiographic visualization. Those skilled in the art will recognize that these

It should be understood that this disclosure is, in many respects, only illustrative. Changes may be made in details, particularly in matters of shape, size, and arrangement of steps without exceeding the scope of the invention. This may include, to the extent that it is appropriate, the use of any of the features of one example embodiment being used in other embodiments. The invention's scope is, of course, defined in the language in which the appended claims are expressed.

## Claims

1. A medical device delivery system (10), comprising:
an outer sheath (12);
an inner catheter (14) disposed within the outer sheath (12), the inner catheter (14) including a plurality of lumens formed therein;
a tubular extension (62) extending distally from a distal end of the inner catheter (14);
wherein the tubular extension (62) is axially movable relative to the inner catheter (14);
a handle (18) attached to the outer sheath (12), wherein the handle (18) is configured to shift the outer sheath (12) between a first position and a second position relative to the inner catheter (14); and
a medical device releasably coupled to the inner catheter (14).
wherein the tubular extension (62) is fixedly attached to a hypotube (60) slidably received within one of the plurality of lumens in the inner catheter.

2. The medical device delivery system of claim 1, further comprising a nose cone (64) attached to the tubular extension (62), in particular wherein the nose cone (64) is fixedly attached at a distal end of the tubular extension (62).

3. The medical device delivery system of any of the preceding claims, wherein the inner catheter (14) is fixed to the handle (18).

4. The medical device delivery system of any of the preceding claims, wherein the tubular extension (62) extends through the medical device.

5. The medical device delivery system of claim 4, further comprising a luer connector (58) attached to a proximal end of the hypotube (60), the luer connector (58) being configured to engage a proximal end of the handle (18).

6. The medical device delivery system of claim 5, wherein the luer connector (58), the hypotube (60), the tubular extension (62), and the nose cone (64) together form a nose cone subassembly having a guidewire lumen extending through the medical device delivery system.

7. The medical device delivery system of any of claims 2-6, wherein the nose cone (64) is axially movable relative to the inner catheter (14) and the handle (18).

8. The medical device delivery system of any of claims 2-7, wherein the nose cone (64) is proximally movable during deployment of the medical device.

9. The medical device delivery system of any of claims 2-8, wherein a proximal portion of the nose cone (64) is configured to abut a distal end of the outer sheath (12) in the first position.

10. The medical device delivery system of any of claims 2-9, wherein the nose cone (64) is spaced distally from the outer sheath (12) in the second position.

11. The medical device delivery system of any of the preceding claims, wherein the medical device is disposed about the tubular extension (62).

12. The medical device delivery system of any of the preceding claims, wherein the tubular extension (62) is axially movable in a proximal direction relative to the inner catheter (14).

13. The medical device delivery system of any of the preceding claims, wherein the handle (18) includes a control knob (112) rotatable about a longitudinal axis of the handle (18), wherein the control knob (112) is operatively connected to the outer sheath (12) such that rotation of the control knob (112) translates the outer sheath (12) axially.

14. The medical device delivery system of any of the preceding claims, wherein the medical device delivery system is configured to deliver and/or deploy a replacement heart valve, a valve repair device or a valvuloplasty device.

## Patentansprüche

1. Zuführsystem (10) für eine medizinische Vorrichtung, umfassend:
eine äußere Hülle (12);
einen innerhalb der äußeren Hülle (12) angeordneten inneren Katheter (14), wobei der innere Katheter (14) eine Mehrzahl darin ausgebildeter Lumen umfasst;
eine rohrförmige Verlängerung (62), die sich distal von einem distalen Ende des inneren Katheters (14) erstreckt;
wobei die rohrförmige Verlängerung (62) relativ zu dem inneren Katheter (14) in axialer Richtung beweglich ist;
einen an der äußeren Hülle (12) befestigten Griff (18), wobei der Griff (18) dazu ausgebildet ist, die äußere Hülle (12) relativ zu dem inneren Katheter (14) zwischen einer ersten Position und einer zweiten Position zu verschieben; und
eine lösbar mit dem inneren Katheter (14) verbundene medizinische Vorrichtung,
wobei die rohrförmige Verlängerung (62) fest mit einem Hypotube (60) verbunden ist, das verschiebbar in einem der mehreren Lumen aufgenommen ist.

2. Zuführsystem für eine medizinische Vorrichtung nach Anspruch 1, ferner umfassend eine an der rohrförmigen Verlängerung (62) befestigter Nasenkonus (64), insbesondere wobei der Nasenkonus (64) fest an einem distalen Ende der rohrförmigen Verlängerung (62) befestigt ist.

3. Zuführsystem für eine medizinische Vorrichtung nach einem der vorhergehenden Ansprüche, wobei der innere Katheter (14) an dem Griff (18) befestigt ist.

4. Zuführsystem für eine medizinische Vorrichtung nach einem der vorhergehenden Ansprüche, wobei sich die rohrförmige Verlängerung (62) durch die medizinische Vorrichtung erstreckt.

5. Zuführsystem für eine medizinische Vorrichtung nach Anspruch 4, ferner umfassend einen an einem proximalen Ende des Hypotubes (60) befestigten Luer-Anschluss (58), wobei der Luer-Anschluss (58) dazu ausgebildet ist, mit einem proximalen Ende des Griffs (18) in Eingriff zu gelangen.

6. Zuführsystem für eine medizinische Vorrichtung nach Anspruch 5, wobei der Luer-Verbinder (58), das Hypotube (60), die rohrförmige Verlängerung (62) und der Nasenkonus (64) zusammen eine Nasenkonusanordnung mit einem sich durch das Zuführsystem für eine medizinische Vorrichtung erstreckendem Führungsdrahtlumen bilden.

7. Zuführsystem für eine medizinische Vorrichtung nach einem der Ansprüche 2-6, wobei der Nasenkonus (64) bezüglich des inneren Katheters (14) und des Griffs (18) axial beweglich ist.

8. Zuführsystem für eine medizinische Vorrichtung nach einem der Ansprüche 2-7, wobei der Nasenkonus (64) während des Einsatzes der medizinischen Vorrichtung proximal beweglich ist.

9. Zuführsystem für eine medizinische Vorrichtung nach einem der Ansprüche 2-8, wobei ein proximaler Abschnitt des Nasenkonus (64) dazu ausgelegt ist, an einem distalen Ende der äußeren Hülle (12) in der ersten Position anzuliegen.

10. Zuführsystem für eine medizinische Vorrichtung nach einem der Ansprüche 2-9, wobei der Nasenkonus (64) in der zweiten Position in distaler Richtung von der äußeren Hülle (12) beabstandet ist.

11. Zuführsystem für eine medizinische Vorrichtung nach einem der vorhergehenden Ansprüche, wobei die medizinische Vorrichtung um die rohrförmige Verlängerung (62) angeordnet ist.

12. Zuführsystem für eine medizinische Vorrichtung nach einem der vorhergehenden Ansprüche, wobei die rohrförmige Verlängerung (62) in einer proximalen Richtung bezüglich des inneren Katheters (14) axial beweglich ist.

13. Zuführsystem für eine medizinische Vorrichtung nach einem der vorhergehenden Ansprüche, wobei der Griff (18) einen Bedienungsknopf (112) aufweist, der um eine Längsachse des Griffs (18) drehbar ist, wobei der Bedienungsknopf (112) funktionsfähig mit der äußeren Hülle (12) verbunden ist, so dass eine Drehung des Bedienungsknopfes (112) die äußere Hülle (12) axial verschiebt.

14. Zuführsystem für eine medizinische Vorrichtung nach einem der vorhergehenden Ansprüche, wobei das Zuführsystem für eine medizinische Vorrichtung zum Freisetzen und/oder Einsetzen einer Ersatzherzklappe, einer Klappenreparaturvorrichtung oder einer Valvuloplastie-Vorrichtung ausgelegt ist.

## Revendications

1. Système d'administration de dispositif médical (10), comprenant :
une gaine externe (12) ;
un cathéter interne (14) disposé à l'intérieur de la gaine externe (12), le cathéter interne (14) comprenant une pluralité de lumières formées à l'intérieur de celui-ci ;
une extension tubulaire (62) s'étendant de manière distale à partir d'une extrémité distale du cathéter interne (14) ;
l'extension tubulaire (62) étant mobile axialement par rapport au cathéter interne (14) ;
un manche (18) fixé à la gaine externe (12), le manche (18) étant configuré pour commuter la gaine externe (12) entre une première position et une seconde position par rapport au cathéter interne (14) ; et
un dispositif médical couplé de manière libérable au cathéter interne (14).
l'extension tubulaire (62) étant fixée de manière fixe à un hypotube (60) logé de manière coulissante dans une lumière parmi la pluralité de lumières du cathéter interne.

2. Système d'administration de dispositif médical selon la revendication 1, comprenant en outre une extrémité conique (64) fixée à l'extension tubulaire (62), en particulier l'extrémité conique (64) étant fixée de manière fixe à une extrémité distale de l'extension tubulaire (62).

3. Système d'administration de dispositif médical selon l'une quelconque des revendications précédentes, le cathéter interne (14) étant fixé au manche (18).

4. Système d'administration de dispositif médical selon l'une quelconque des revendications précédentes, l'extension tubulaire (62) s'étendant à travers le dispositif médical.

5. Système d'administration de dispositif médical selon la revendication 4, comprenant en outre un raccord luer (58) fixé à une extrémité proximale de l'hypotube (60), le raccord luer (58) étant configuré pour venir en prise avec une extrémité proximale du manche (18).

6. Système d'administration de dispositif médical selon la revendication 5, le raccord luer (58), l'hypotube (60), l'extension tubulaire (62) et l'extrémité conique (64) formant ensemble un sous-ensemble d'extrémité conique ayant une lumière de fil de guidage s'étendant à travers le système d'administration de dispositif médical.

7. Système d'administration de dispositif médical selon l'une quelconque des revendications 2 à 6, l'extrémité conique (64) étant mobile axialement par rapport au cathéter interne (14) et au manche (18).

8. Système d'administration de dispositif médical selon l'une quelconque des revendications 2 à 7, l'extrémité conique (64) étant mobile de manière proximale pendant le déploiement du dispositif médical.

9. Système d'administration de dispositif médical selon l'une quelconque des revendications 2 à 8, une partie proximale de l'extrémité conique (64) étant configurée pour venir en butée contre une extrémité distale de la gaine externe (12) dans la première position.

10. Système d'administration de dispositif médical selon l'une quelconque des revendications 2 à 9, l'extrémité conique (64) étant espacée de manière distale de la gaine externe (12) dans la seconde position.

11. Système d'administration de dispositif médical selon l'une quelconque des revendications précédentes, le dispositif médical étant disposé autour de l'extension tubulaire (62).

12. Système d'administration de dispositif médical selon l'une quelconque des revendications précédentes, l'extension tubulaire (62) étant mobile axialement dans une direction proximale par rapport au cathéter interne (14).

13. Système d'administration de dispositif médical selon l'une quelconque des revendications précédentes, le manche (18) comprenant un bouton de commande (112) pouvant tourner autour d'un axe longitudinal du manche (18), le bouton de commande (112) étant relié fonctionnellement à la gaine externe (12) de sorte que la rotation du bouton de commande (112) déplace en translation axialement la gaine externe (12).

14. Système d'administration de dispositif médical selon l'une quelconque des revendications précédentes, le système d'administration de dispositif médical étant configuré pour administrer et/ou déployer une valvule cardiaque de remplacement, un dispositif de réparation de valvule ou un dispositif de valvuloplastie.
